# EUROPEAN PATENT APPLICATION

(11) **EP 3 185 591 A1**
(43) Date of publication of application: **28.06.2017**
(21) Application number: 14881758.8
(22) Date of filing: 12.12.2014
(51) Int. Cl.: H04W 4/00

(54) **METHOD AND DEVICE FOR EXCHANGING INFORMATION BETWEEN SMART TERMINAL AND WEARABLE DEVICE**

(30) Priority: 19.08.2014 CN 201410409343
(71) Applicant: ZTE Corporation, Shenzhen, Guangdong 518057 (CN)
(72) Inventor: SUN, Aifang, Shenzhen Guangdong 518057 (CN)
(74) Representative: Winter, Brandl, Fürniss, Hübner, Röss, Kaiser, Polte - Partnerschaft mbB
(86) International application number: PCT/CN2014/093754
(87) International publication number: WO 2015/117503

(57) **Abstract**

The present invention provides a method and an apparatus for interacting information between a smart terminal and a wearable device. The method includes: acquiring first control information containing behavior information of a tester; acquiring second control information containing brain wave information of the tester; comparing the first control information with the second control information to obtain a comparison result; and executing the first control information when the comparison result indicates that the first control information is consistent with the second control information. In the above-mentioned solution, a control signal containing the behavior information of the tester is compared with a control signal containing the brain wave information of the tester, and a corresponding operation can be performed only if the two control signals are consistent with each other. In this way, misoperation is avoided effectively, and an effective communication between the wearable device and the mobile terminal is ensured.

## Description

### TECHNICAL FIELD

The present invention relates to the field of wireless communication, in particular to a method and an apparatus for interacting information between a smart terminal and a wearable device.

### BACKGROUND

With a development of science and technology, smart devices also spring up like mushrooms. With an emergence of high-performance smart phones, tablet computers, smart bracelets and smart glasses, an increasing number of hardware manufacturers focus on "smart" now. In a market environment in which the smart devices are increasingly popular, benefited from a development of a chip technology and Internet, the wearable device, which is a portable smart device that can be directly worn on a body of a user or be integrated into clothes or accessories of the user, arises. Neither the smart bracelets nor the smart glasses are merely hardware devices, and further realize powerful functions through software support, data interaction and cloud interaction, so as to bring convenience to our lives.

With a successive introduction of the wearable device such as the smart bracelet, a Bluetooth scale, a smart watch and smart glasses, people pay more and more attentions on an interaction between a smart phone terminal and the wearable device. When the information interaction is performed between the wearable device and the smart terminal, regarding how to ensure reliability and accuracy of acquired information data, how to ensure that the acquired information data can be transmitted to the smart terminal securely and how to ensure privacy of the acquired data information, a series of problems also arise. Moreover, it is an urgent problem to be solved that communication efficiency between the wearable device and the smart terminal is reduced and communication resources are wasted due to misoperation of the wearable device.

### SUMMARY

Embodiments of the present invention provide a method and an apparatus for interacting information between a smart terminal and a wearable device, so as to solve at least the problem that communication efficiency between the wearable device and the mobile terminal is reduced and communication resources are wasted due to misoperation of the wearable device in the existing art.

In order to solve the above technical problems, embodiments of the present invention provide a method for interacting information between a smart terminal and a wearable device, including:
acquiring first control information containing behavior information of a tester;
acquiring second control information containing brain wave information of the tester;
comparing the first control information with the second control information to obtain a comparison result; and
executing the first control information when the comparison result indicates that the first control information is consistent with the second control information.

Further, before comparing the first control information with the second control information to obtain a comparison result, the method further includes:
performing security authentication on the first control information and the second control information respectively.

Further, the method further includes: storing the first control information when the comparison result indicates that the first control information is consistent with the second control information.

Further, the method further includes:
performing a control to resume acquiring the first control information when the comparison result indicates that the first control information is inconsistent with the second control information.

Further, the behavior information includes a body action and/or language of the tester.

Embodiments of the present invention provide an apparatus for interacting information between a smart terminal and a wearable device, including:
a first acquisition module, arranged to acquire first control information containing behavior information of a tester;
a second acquisition module, arranged to acquire second control information containing brain wave information of the tester;
a comparison module, arranged to compare the control information with the second control information to obtain a comparison result; and
an execution module, arranged to execute the first control information when the comparison result indicates that the first control information is consistent with the second control information.

Further, the apparatus further includes:
a security authentication module, arranged to perform security authentication on the first control information and the second control information respectively.

Further, the apparatus further includes:
a storage module, arranged to store the first control information when the comparison result indicates that the first control information is consistent with the second control information.

Further, the apparatus further includes:
a control module, arranged to control the first acquisition module to resume acquiring the first control information when the comparison result indicates that the first control information is inconsistent with the second control information.

Advantageous effects of the present invention lie in: according to the above-mentioned solution, a control signal containing the behavior information of the tester is compared with a control signal containing the brain wave information of the tester, and a corresponding operation can be performed only if the above two control signals are consistent with each other. In this way, the misoperation is avoided effectively, and an effective communication between the wearable device and the mobile terminal is ensured.

### BRIEF DESCRIPTION OF DRAWINGS

Fig. 1 is a schematic flow diagram illustrating a method according to a first embodiment of the present invention;
Fig. 2 is a schematic flow diagram illustrating a method according to a second embodiment of the present invention;
Fig. 3 is a schematic modular diagram illustrating an apparatus according to embodiments of the present invention;
Fig. 4 is an architecture diagram illustrating information interaction between a smart terminal and a wearable device according to embodiments of the present invention;
Fig. 5 is a schematic diagram illustrating a behavior management module according to embodiments of the present invention; and

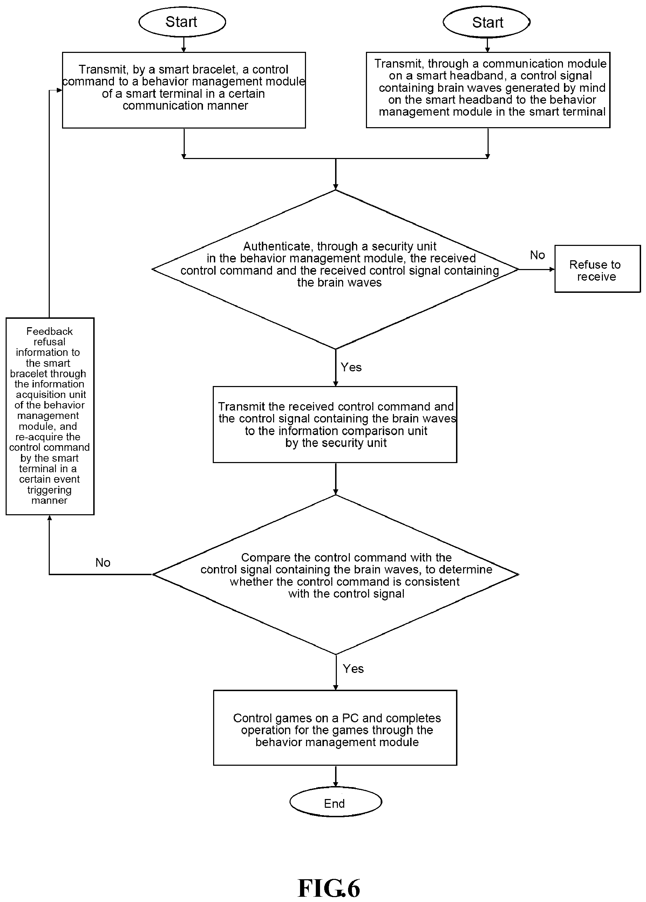

Fig. 6 is a flow chart illustrating information interaction between a smart bracelet/ smart headband and the smart terminal according to embodiments of the present invention.

### DETAILED DESCRIPTION

In order to make objectives, technical solutions and advantages of the present invention clearer, the present invention is described in details below in combination with drawings and specific embodiments.

With respect to the problem that communication efficiency between the wearable device and the mobile terminal is reduced and communication resources are wasted due to misoperation of the wearable device, the present invention provides a method and an apparatus for interacting information between a smart terminal and a wearable device.

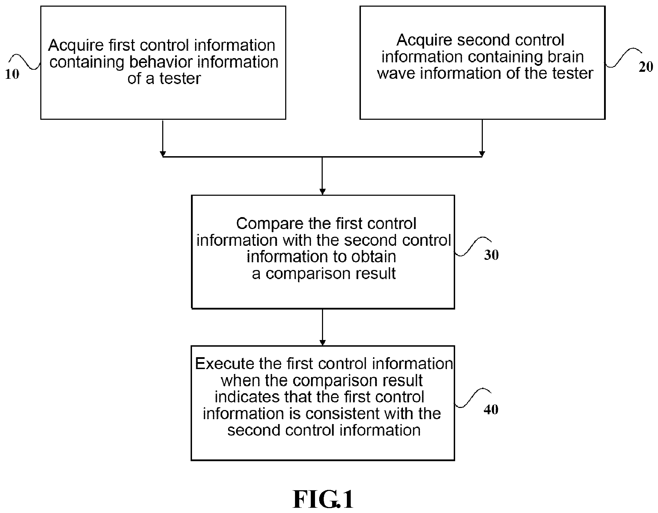

As shown in Fig. 1, a first embodiment of the present invention provides a method for interacting information between the smart terminal and the wearable device, including the following steps:
step 10, first control information containing behavior information of a tester is acquired;
step 20, second control information containing brain wave information of the tester is acquired;
step 30, the first control information is compared with the second control information, so as to obtain a comparison result; and
step 40, the first control information is executed when the comparison result indicates that the first control information is consistent with the second control information.

According to the above-mentioned embodiment, a control signal containing the behavior information of the tester is compared with a control signal containing the brain wave information of the tester, and a corresponding operation can be performed only if the two control signals are consistent with each other. In this way, the misoperation is avoided effectively, and the effective communication between the wearable device and the mobile terminal is ensured.

It should be noted that the behavior information includes body actions and/or language of the tester, and the second control information refers to information contained in brain waves formed by mind of the tester.

It should be noted that the step 10 and the step 20 are performed simultaneously.

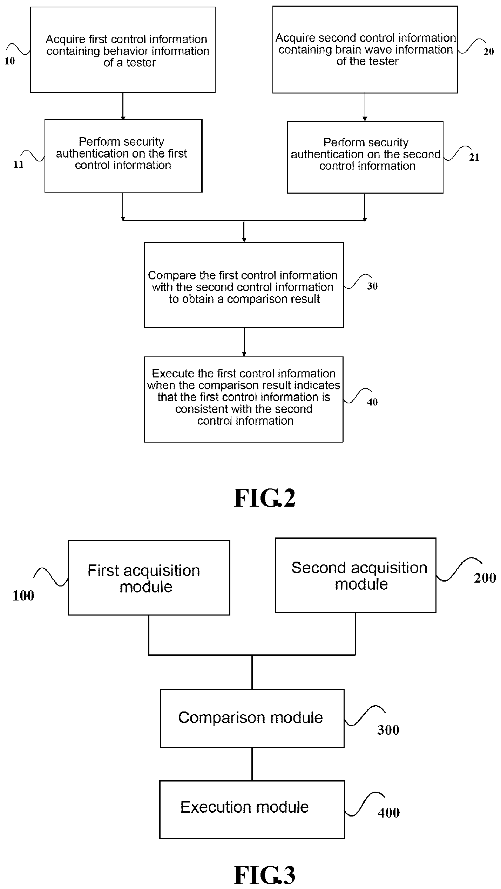

In order to ensure that the acquired first control information and the second control information are legal information, as shown in Fig. 2, the method according to a second embodiment of the present invention further includes the following steps before step 30:
step 11, security authentication is performed on the first control information; and
step 21, the security authentication is performed on the second control information.

It should be noted that the security authentication is performed to ensure accuracy and reliability of the acquired first control information and the second control information, i.e., the smart terminal only receives information transmitted by the wearable device with which connection is established. It should be noted herein that a function of the security authentication is the same as an implementation principle of Bluetooth in the existing art, and is not described in details herein.

In another embodiment of the present invention, the method further includes a step that the first control information is stored when the comparison result indicates that the first control information is consistent with the second control information.

It should be noted that the first control information is stored to facilitate the subsequent use of the first control information.

The method according to the embodiment of the present invention further includes a following step: it is controlled to resume acquiring the first control information when the comparison result indicates that the first control information is inconsistent with the second control information.

It should be noted that, when the first control information is inconsistent with the second control information, it indicates that the behavior information represented by the first control information is misoperation of the tester or false unrecognizable information transmitted. Then, the smart terminal may resume acquiring the first control information in a signal triggering manner. It should be noted herein that, the first control information is not acquired without restriction, and when a preset number of acquisition has been dome (in which the preset number can be set as needed, e.g., two, three and the like), the acquisition for the first control information shall be stopped, for a purpose of avoiding excessive consumption of communication resources.

According to the above-mentioned embodiment, the mind of the tester is compared with behavior of the tester, and the corresponding operation can be realized only if the behavior is consistent with the mind. In this way, the misoperation of the tester is avoided effectively, and the effective communication between the wearable device and the mobile terminal is ensured.

As shown in Fig. 3, an embodiment of the present invention provides an apparatus for interacting information between a smart terminal and a wearable device, including:
a first acquisition module 100, arranged to acquire first control information containing behavior information of a tester;
a second acquisition module 200, arranged to acquire second control information containing brain wave information of the tester;
a comparison module 300, arranged to compare the control information with the second control information to obtain a comparison result; and
an execution module 400, arranged to execute the first control information when the comparison result indicates that the first control information is consistent with the second control information.

Specifically, the apparatus further includes:
a security authentication module, arranged to perform security authentication on the first control information and the second control information respectively.

Specifically, the apparatus further includes:
a storage module, arranged to store the first control information when the comparison result indicates that the first control information is consistent with the second control information.

Specifically, the apparatus further includes:
a control module, arranged to control the first acquisition module 100 to resume acquiring the first control information when the comparison result indicates that the first control information is inconsistent with the second control information.

It should be noted that, an embodiment of the apparatus is an apparatus corresponding to the above-mentioned method, all implementation modes of the above-mentioned method are applicable to the embodiment of the apparatus, and technical effects same as those of the above-mentioned method can also be achieved.

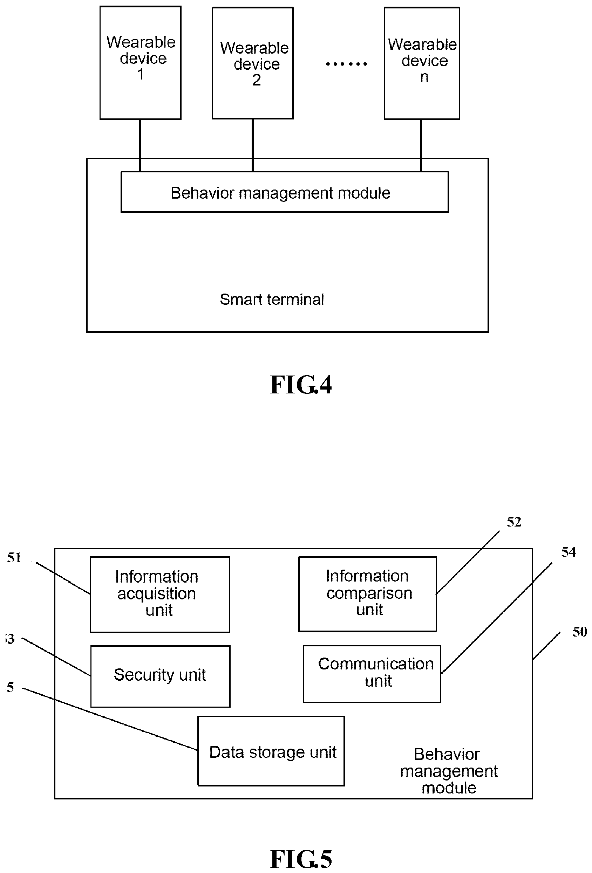

As shown in Fig. 4 and Fig. 5, the wearable device implements information interaction with the smart terminal through a behavior management module 50 provided on the smart terminal. The behavior management module 50 mainly includes an information acquisition unit 51, an information comparison unit 52, a security unit 53, a communication unit 54 and a data storage unit 55.

The information acquisition unit 51 is arranged to acquire information data including behavior information such as language and body actions, and brain wave information.

The information comparison unit 52 is arranged to compare brain waves formed by mind of the tester with the acquired behavior information of the tester, so as to ensure reliability and security of the behavior information acquired by the wearable device. A concrete implementation method is as follows: when the comparison result of the brain waves and the behavior information is consistency, it is proved that the acquired behavior information is accurate information desired by the tester, and the behavior information is transmitted to the data storage module for storage; otherwise, the information acquisition unit 51 will reissue an information acquisition request for acquiring information; the number of this request includes but not limited to 2~3; and after acquiring again, it is determined whether to receive or refuse the acquired information data according to an information request.

The security unit 53 is arranged to perform security acquisition and security transmission on the information data for the smart terminal, while securing privacy of data.

The security transmission of the information data means that the information acquired by the wearable device can be transmitted to the smart terminal securely, by adopting an authentication or encryption mode including but not limited to that negotiated by manufacturers or providers of the wearable device and the smart terminal together. More specifically, the interacted data information is encrypted in a certain encryption mode, and legitimacy of the data information is determined through authentication and certification by both the wearable device and the smart terminal after the data information being received by the wearable device or the smart terminal. Further, the authentication mode may adopt a mode of being compared with message of the smart terminal for issuing the request, or the wearable device determines the legitimacy of requested information data by comparing the requested information data with terminal technical parameters of a built-in preset mobile terminal. The privacy of the information data is protected, which means that the wearable device determines whether the information data requested by the smart terminal is legal through presetting. If the requested information exceeds a provided range, the wearable device can refuse to transmit the data to the smart terminal, and the smart terminal fails to request the data. Otherwise, the wearable device transmits the data to the smart terminal securely, so as to meet requirements of the user of the smart terminal.

The communication unit 54 is a communication unit used by the smart terminal to communicate with the wearable device, and includes but not limited to use of a wireless communication technology such as WIFI or the 3rd Generation Telecommunication (3G).

The data storage unit 55 is arranged to store the information data authenticated by the security unit 53 and processed by the information comparison unit 52.

Through the behavior management module 50, the information can be transmitted between the wearable device and the smart terminal, and the reliability and the security of the acquired data can be ensured, which are necessary for the information interaction between the wearable device and the smart terminal.

An implementation process of the present invention is described in details by taking a following situation as an example, in which a smart bracelet and a smart headband control games on a personal computer (PC) through the smart terminal.

A control command is transmitted, by the smart terminal, to the behavior management module of the smart terminal in a certain communication manner (including but not limited to communication manners such as WIFI, Bluetooth, mobile communication and the like), and is acquired through the information acquisition unit of the behavior management module. Meanwhile, a control signal containing brain waves generated by the mind on the smart headband is transmitted, through a communication module on the smart headband, to the information acquisition unit in the behavior management module in the smart terminal.

After being received from the smart bracelet by the information acquisition unit in the behavior management module, the control command is subjected to a bidirectional authentication and certification between the smart terminal and the smart bracelet through the security unit firstly. The certification manner is implemented by being compared with the information data of the smart bracelet preset in the data storage unit of the behavior management module. The information data of the smart bracelet preset in the data storage unit includes a type of the smart bracelet, an identification number of the smart bracelet preset in the data storage unit, and the like. The control command is transmitted to the information comparison unit by the security unit if the control command passes the authentication, otherwise, the control command is refused to be received.

It should be noted that, the bidirectional authentication and certification between the smart terminal and the smart headband is similar to that between the smart terminal and the smart bracelet, i.e., the certification manner is implemented by being compared with information data of the smart headband preset in the data storage unit. The information data of the smart headband preset in the data storage unit includes a type of the smart headband, an identification number of the smart headband preset in the data storage unit, and the like. If information parameters of the smart headband received by the security unit are consistent with preset information parameters, the authentication and certification are passed, and the control command is transmitted to the information comparison unit by the security unit. Otherwise, a brain wave signal containing the control signal is refused to be received.

Then, the information comparison unit compares the received control command with the control signal containing the brain waves. If the control command is consistent with the control signal, the control command is determined as correct information data, and the control command can directly control the games on the PC through the behavior management module in an event driving manner to complete operations for the games. Otherwise, the smart terminal feeds back refusal information to the smart bracelet through the information acquisition unit of the behavior management module, and re-acquires the control command in an event triggering manner.

As a preferred embodiment of the present invention, a computer-readable medium having a program code executable by a processor is provided, and when being executed, the program code enables the processor to perform the following steps:
step 1, first control information containing behavior information of a tester is acquired;
step 2, second control information containing brain wave information of the tester is acquired;
step 3, the first control information is compared with the second control information to and obtain a comparison result; and
step 4, the first control information is executed when the comparison result indicates that the first control information is consistent with the second control information.

As another preferred embodiment of the present invention, an apparatus for interacting information between a smart terminal and a wearable device is provided. The apparatus includes: a processor, arranged to acquire first control information containing behavior information of a tester and second control information containing brain wave information of the tester; compare the first control information with the second control information to obtain a comparison result; and execute the first control information when the comparison result indicates that the first control information is consistent with the second control information.

It should be noted that, according to the present invention, the misoperation on the wearable device by the tester is effectively avoided, and the effective communication between the wearable device and the mobile terminal is ensured.

The above are preferred embodiments of the present invention. It should be noted that those ordinary skilled in the art can also perform various modifications and improvements without departing from principles of the present invention, and these modifications and improvements are also included in a protection scope of the present invention.

### Industrial Applicability

As described above, the method and the apparatus for interacting information between the smart terminal and the wearable device provided by embodiments of the present invention have the following advantageous effects: the control signal containing the behavior information of the tester is compared with the control signal containing the brain wave information of the tester, and a corresponding operation can be performed only if the two control signals are consistent with each other; and in this way, the misoperation is avoided effectively, and the effective communication between the wearable device and the mobile terminal is ensured.

## Claims

1. A method for interacting information between a smart terminal and a wearable device, comprising:
acquiring first control information containing behavior information of a tester;
acquiring second control information containing brain wave information of the tester;
comparing the first control information with the second control information to obtain a comparison result; and
executing the first control information when the comparison result indicates that the first control information is consistent with the second control information.

2. The method according to claim 1, further comprising the following before comparing the first control information with the second control information to obtain a comparison result:
performing security authentication on the first control information and the second control information respectively.

3. The method according to claim 1, further comprising: storing the first control information when the comparison result indicates that the first control information is consistent with the second control information.

4. The method according to claim 1, further comprising:
performing a control to resume acquiring the first control information when the comparison result indicates that the first control information is inconsistent with the second control information.

5. The method according to claim 1, wherein the behavior information comprises body actions and/or language of the tester.

6. An apparatus for interacting information between a smart terminal and a wearable device, comprising:
a first acquisition module, arranged to acquire first control information containing behavior information of a tester;
a second acquisition module, arranged to acquire second control information containing brain wave information of the tester;
a comparison module, arranged to compare the control information with the second control information to obtain a comparison result; and
an execution module, arranged to execute the first control information when the comparison result indicates that the first control information is consistent with the second control information.

7. The apparatus according to claim 6, further comprising:
a security authentication module, arranged to perform security authentication on the first control information and the second control information respectively.

8. The apparatus according to claim 6, further comprising:
a storage module, arranged to store the first control information when the comparison result indicates that the first control information is consistent with the second control information.

9. The apparatus according to claim 6, further comprising:
a control module, arranged to control the first acquisition module to resume acquiring the first control information when the comparison result indicates that the first control information is inconsistent with the second control information.

10. A computer-readable medium having a program code executable by a processor, the program code, when being executed, enables the processor to perform the following steps:
acquiring first control information containing behavior information of a tester;
acquiring second control information containing brain wave information of the tester;
comparing the first control information with the second control information to obtain a comparison result; and
executing the first control information when the comparison result indicates that the first control information is consistent with the second control information.

11. An apparatus for interacting information between a smart terminal and a wearable device, comprising a processor, wherein the processor is arranged to acquire first control information containing behavior information of a tester, and second control information containing brain wave information of the tester; compare the first control information with the second control information to obtain a comparison result, and execute the first control information when the comparison result indicates that the first control information is consistent with the second control information.
